# EUROPÄISCHE PATENTSCHRIFT

(11) **EP 2 151 510 B1**
(45) Veröffentlichungstag und Bekanntmachung des Hinweises auf die Patenterteilung: **31.12.2014**
(21) Anmeldenummer: 09009235.4
(22) Anmeldetag: 15.07.2009
(51) Int. Cl.: C23C 16/509, C23C 16/54, C23C 16/04, A61L 2/14

(54) **Vorrichtung und Verfahren zur Plasmabehandlung von Hohlkörpern**
Device and method for plasma treating hollow bodies
Dispositif et procédé destinés au traitement plasma de corps creux

(30) Priorität: 08.08.2008 DE 102008037159
(43) Veröffentlichungstag der Anmeldung: 10.02.2010
(73) Patentinhaber: Krones AG, 93073 Neutraubling (DE)
(72) Erfinder: Krüger, Jochen, Dr., 93095 Hagelstadt (DE); Felts, John, Alameda CA 94501 (US)
(74) Vertreter: Grünecker, Kinkeldey, Stockmair & Schwanhäusser

(56) Entgegenhaltungen:
- EP-A- 1 548 149
- DE-A1- 10 134 037
- JP-A- 61 019 775
- US-A1- 2006 150 909

## Beschreibung

Die Erfindung bezieht sich auf eine Vorrichtung und ein Verfahren zur Plasmabehandlung von Hohlkörpern. Insbesondere dient die erfindungsgemäße Vorrichtung zur Durchführung eines PECVD-Verfahrens (PECVD = plasma enhanced chemical vapor deposition) bei reduziertem Druck, bei dem dünne Schichten auf der inneren Oberfläche der Hohlkörper abgeschieden werden.

Als Hohlkörper sind insbesondere Behälter zur Verpackung von Lebensmitteln, Getränken oder Arzneimitteln umfasst. Die Behälter können Kunststoffbehälter aus z.B. Polyester, insbesondere PET, PS, PP oder PE sein. Weiter können biologisch hergestellte und/oder biologisch abbaubare Kunststoffe verwendet werden. Die Erfindung umfasst die Plasmabehandlung aller gängigen Behältertypen, insbesondere zwischen 0,1 und 10 I. Die Herstellung der Behälter kann durch alle gängigen Formgebungsarten, wie z.B. Streckblasen, Extrusion, Spritzguss, erfolgen.

Die Schicht kann eine glasartige SiOx-Schicht sein. Der Wert von x liegt insbesondere zwischen 1.5 und 2.5. Die Generierung der Schicht erfolgt üblicherweise durch die Oxidation von Siloxan und/oder Silazan Precursor Molekülen, insbesondere HMDSO, Hexamethyldisiloxan, mit Sauerstoff durch Ionisation im Plasma und Rekombination. Sauerstoff wird dabei bevorzugt im Überschuss eingesetzt. Weiter können kohlenstoffhaltige Schichten, insbesondere sogenannte DLC (Diamond like Carbon) Schichten aufgebracht werden. Als Precursor Molekül wird hier insbesondere Acetylen eingesetzt.

Die Plasmabeschichtung der Hohlkörper dient insbesondere dazu, die Permeabilität von Kunststoffflaschen, insbesondere PET-Getränkeflaschen gegenüber Gasen wie Sauerstoff und/oder Kohlendioxid zu erniedrigen. Herkömmliche Kunststofftypen besitzen für Sauerstoff-sensible und/oder karbonisierte Getränke, wie z.B. Bier, Saft, Milch eine ungenügende Gasbarriere, so dass die resultierende Produkthaltbarkeit zu niedrig ist. Beschichtete PET-Flaschen besitzen üblicherweise im Vergleich zu unbeschichteten Referenzflaschen einen BIF (Barrier Improvement Factor) von insbesondere ca. 5 (Sauerstoff) bzw. ca. 2-3 (Kohlendioxid), d.h. besitzen eine um diesen Faktor vergrößerte Barriere. Dadurch ist eine erhebliche Verlängerung der Produkthaltbarkeit erzielbar.

Eine weitere Anwendung von plasmabeschichteten PET-Flaschen in der Getränkeindustrie besteht darin, dass Schadstoffe und/oder Verunreinigungen, die sich im PET befinden, wie z.B. Acetaldehyd, effektiv davon abgehalten werden, durch die Schicht zu permeieren, und ins Flascheninnere zu gelangen. Somit können behandelte PET-Flaschen für Getränke ohne Eigengeschmack, wie insbesondere stilles Wasser verwendet werden.

Ein weiterer Vorteil von beschichteten Hohlkörpern besteht darin, dass Reinigungsmittel und/oder Sterilisationsmittel, wie z.B. H₂O₂ oder Seifen, nicht in die Hohlkörperwand migrieren können, und so nach Befüllung der Hohlkörper nicht in das Produkt gelangen können. Weiter besteht der Vorteil, dass organische Füllgüter/Verunreinigungen nicht in die Flaschenwand migrieren, und so ein Wiederverwenden der Hohlkörper z.B. für die Lebensmittelverpackung ermöglichen.

Neben der Plasmabeschichtung ist insbesondere die Plasmasterilisation zum Reinigen von Hohlkörpern, bevorzugt im Lebensmittel- oder Getränkeverpackungsbereich, möglich. Die Sterilisierung der Hohlkörper über ein Plasma erfolgt durch das Einwirken von kurzwelliger elektromagnetischer Strahlung im UV Bereich, sowie ionisierten und radikalen Plasmakomponenten, auf Schadstoffe, wie z.B. schädliche Mikroorganismen. Die Plasmasterilisation kann alternativ zur Plasmabeschichtung durchgeführt werden. Beide Verfahren können auch in Kombination während der Behandlung eines Hohlkörpers durchgeführt werden.

Vorrichtungen und Verfahren zur Plasmabehandlung von Behältern, insbesondere zur Herstellung von plasmabeschichteten Kunststoffbehältern zur Verbesserung der Gasbarriere sind allgemein bekannt.

Die 102 25 609 A1 zeigt ein Verfahren, bei dem das Innere eines Werkstücks in einer evakuierbaren Einzelkammer einer Plasmabeschichtung unterzogen wird, bei der das Plasma unter Verwendung von Mikrowellenenergie generiert wird.

Ein weiteres Verfahren zur Innenbeschichtung von insbesondere PET-Flaschen wird in US 2002/0179603 A1 beschrieben. Dabei wird eine kohlenstoffhaltige Beschichtung auf die Innenseite der Flaschen über ein Plasmaverfahren aufgebracht, bei dem die zu beschichtende Flasche in eine Einzelkammer eingebracht wird, und im Inneren der Flasche ein Plasma mittels Mikrowellenenergie erzeugt wird.

Die im Stand der Technik offenbarten Verfahren haben den Nachteil, dass die Plasmabehandlung der Hohlkörper in Einzelkammern erfolgt, in denen sich jeweils ein Hohlkörper befindet. Dies bedingt bei einer großtechnischen Umsetzung, bei der eine hohe Zahl beschichteter Behälter in möglicht kurzer Zeit hergestellt werden soll, eine Vielzahl solcher Einzelkammern. Dadurch sind solche Vorrichtungen und Verfahren aus dem Stand der Technik mit hohen Kosten und großen Anlagendimensionen verbunden.

Ein Ansatz, diese Nachteile zu umgehen, ist in DE 10 2004 03 6063 A1 beschrieben. Darin ist eine Vorrichtung und ein Verfahren zur Plasmabeschichtung und/oder Sterilisation offenbart, bei dem in einer Vakuumkammer über eine stabförmige Elektrode im Inneren von Behältern ein Plasma mittels Mikrowellenenergie generiert wird.

Mikrowellengenerierte Plasmen, d.h. Plasmen, die durch elektromagnetische Wellen im GHz Bereich erzeugt werden, haben allgemein den Nachteil, dass es schwierig ist, eine einheitliche Plasmabehandlung der Substrate, insbesondere im Falle von Hohlkörpern, zu erzielen. D.h. es ist insbesondere schwierig, die Abscheidung einer einheitlichen Schicht mit einer guten Gasbarriere zu gewährleisten. Weiter ist im Falle von mikrowellengenerierten Plasmen ein hoher Energieeintrag erforderlich, was aus Kostengesichtspunkten ungünstig ist.

Zur Umgehung dieser Nachteile gibt es Vorschläge, das Plasma über die Einbringung von Hochfrequenzenergie, d.h. elektromagnetischen Wellen, im kHz- bis MHz-Bereich, zu generieren. Dies wird z.B. in US 6,180,191 B1, US 6,539,890 B1, oder US 6,112,695 beschrieben. Allerdings ist auch in diesen Fällen eine Einzelkammer beschrieben, in der sich die Behälter während der Plasmabehandlung befinden.

Die DE 101 34 037 A1 beschreibt eine Vorrichtung zur Plasmabehandlung von Gefäßen nach dem Oberbegriff des Anspruchs 1.

Die JP 61 019775 A beschreibt eine Vorrichtung, in der röhrenförmige Werkstücke aus Metall in einer ringförmigen Unterdruckkammer zwischen Heizstäben erwärmt und von innen mit Hilfe röhrenförmiger, Prozessgas führender Elektroden plasmabeschichtet werden. Dabei stehen die Werkstücke auf geerdeten Auflagetellern und wirken als Gegenelektroden.

Die US 2006/150909 A1 beschreibt eine Rundläufermaschine zur Plasmabeschichtung von Flaschen, die jeweils paarweise in Einzelkammern eingebracht und über zu den Flaschen koaxial ausgerichtete und den jeweiligen Flaschenböden gegenüberliegende Elektroden mit Mikrowellenstrahlung beaufschlagt werden.

Die EP 1 548 149 A beschreibt die Verwendung stabförmiger Elektroden im Inneren eines zu behandelnden Gefäßes zur Plasmaabscheidung von SiOx.

Somit sind die im Stand der Technik beschriebenen Vorrichtungen und Verfahren kompliziert, aufwändig, bzw. nur bedingt für einen großtechnischen Prozess geeignet, da sie hohe Anlagekosten, große Anlagenausmaße, sowie eine geringe Anlageneffizienz aufweisen.

Die Aufgabe der Erfindung liegt in der Bereitstellung einer Vorrichtung und eines Verfahrens zur Plasmabehandlung von Hohlkörpern, die im Vergleich zu herkömmlichen Vorrichtungen und Verfahren einen kompakten, vereinfachten und kostengünstigen Aufbau aufweisen, und eine möglichst gleichmäßige und einheitliche Plasmabehandlung erlauben.

Diese Aufgaben werden mit einer Vorrichtung nach Anspruch 1 erfindungsgemäß dadurch gelöst, dass die Mittel zum Erzeugen des Plasmas eine im Querschnitt im wesentlichen U-förmige Elektrode umfassen, die in der Unterdruckbehandlungskammer angeordnet ist, wobei die Vorrichtung so eingerichtet ist, dass die Hohlkörper während der Durchführung der Plasmabehandlung zumindest teilweise in die U-förmige Elektrode eintauchen, und zumindest zeitweise relativ zur U-förmigen Elektrode bewegt werden.

Die U-förmige Elektrode hat einen im Wesentlichen U-förmigen Querschnitt. Die Radien sind erfindungsgemäß frei wählbar. Insbesondere ist eine Ausführungsform umfasst, die im Wesentlichen gerade Seitenwände aufweist, und die beiden seitlichen Schenkel im rechten Winkel zur Bodenwand angeordnet sind.

Alternativ erfolgt eine Anpassung des Elektrodenquerschnitts an die Hohlkörpergeometrie, d.h. die Form der U-förmigen Elektrode ist im Wesentlichen der Form der Hohlkörper angepasst.

Die U-förmige Elektrode ist in Längsrichtung tunnelartig ausgedehnt. Dadurch ist es möglich, mehrere Hohlkörper gleichzeitig im Inneren der Elektrode unterzubringen. Somit kann auf mehrere separate Einzelkammern verzichtet werden. Gleichzeitig ist es so möglich den Zwischenraum, d.h. den Abstand zwischen den Hohlkörpern zu erniedrigen. Dadurch ist eine platzsparende Anordnung, d.h. kleinere Bauraumausmaße, möglich.

Bevorzugt ist die U-förmige Elektrode austauschbar in der Unterdruckbehandlungskammer angeordnet. Dadurch ist es möglich, für verschiedene Hohlkörpergeometrien unterschiedliche U-förmige Elektroden zu verwenden. Dadurch ist eine variable und effektive Verfahrensführung und eine möglichst einheitliche Plasmabehandlung der Höhlkörper ermöglicht, da die Art der U-förmigen Elektrode jeweils an unterschiedliche Hohlkörpergeometrien angepasst werden kann

Die Größe und Tiefe der U-förmigen Elektrode ist so gestaltet, dass der Zwischenraum zwischen den Hohlkörpern und der U-förmige Elektrode möglichst gering ist, und die Hohlkörper möglichst ganz in den Innenraum der U-förmigen Elektrode eingeführt werden können. Die U-förmige Elektrode so ausgelegt, dass der Großteil der herkömmlichen Getränkeflaschen (d.h. Volumen von ca. 0,1 l bis 10 l) damit behandelt werden können.

Nach einer bevorzugten Ausgestaltung sind die Seitenwände der U-förmigen Elektrode parallel zur rohrförmigen Gegenelektrode angeordnet. Besonders bevorzugt ist eine Anordnung, in der sich der Abstand der rohrförmigen Gegenelektrode zu den Seitenwänden der U-förmigen Elektrode im Wesentlichen gleich ist. Dadurch ist ein einheitliches elektromagnetisches Feld im Inneren der U-förmigen Elektrode generierbar, so dass eine möglichst einheitliche Plasmabehandlung der Hohlkörper erfolgt.

Bevorzugt besteht die U-förmige Elektrode im Wesentlichen aus einem elektrisch leitenden Material, insbesondere aus Kupfer oder Edelstahl.

Nach einer vorteilhaften Ausgestaltung der Erfindung umfasst die Vorrichtung mindestens zwei separate Absaugvorrichtungen, wobei eine Absaugvorrichtung im Inneren des Hohlkörpers einen ersten reduzierten Druck (P2) generiert, und eine andere Absaugvorrichtung im Inneren der Unterdruckbehandlungskammer, aber außerhalb der Hohlkörper, einen zweiten reduzierten Druck (P1) generiert, wobei der erste Druck (P2) geringer, bevorzugt mindestens 10 bis 2000 mal geringer als der zweite Druck (P1) ist. Während der Durchführung der Plasmabehandlung ist das Innere der Hohlkörper gasdicht von der Unterdruckbehandlungskammer abgeschlossen. Insbesondere ist bevorzugt, dass der Druck (P2) ca. 2 Pa ist, während der Druck (P1) ca. 3500 Pa ist. Dadurch wird gewährleistet, dass das Plasma ausschließlich im Inneren der Hohlkörper generiert wird, und so eine Plasmabehandlung ausschließlich im Hohlkörperinneren stattfindet. Über die Einstellung der oben beschriebenen Druckverhältnisse ist es möglich, gezielt nur dort ein Plasma zu generieren, wo eine Plasmabehandlung erwünscht ist. Dadurch kann die Vorrichtung energetisch kostengünstig betrieben werden. Die Ausführungsform mit mindestens zwei separaten Pumpensystemen hat weiter den Vorteil, dass die Vorrichtung wartungs- und störungsunanfälliger arbeitet, da im Vergleich zu Vorrichtungen, die lediglich ein Pumpensystem besitzen, weniger Ventile zum Generieren der unterschiedlichen Drücke (P1) und (P2) nötig sind.

Nach einer weiteren Ausgestaltung umfassen die Mittel zum Erzeugen des Plasmas einen Generator zum Erzeugen eines elektromagnetischen Feldes im Hochfrequenzbereich. Durch die Erzeugung eines elektromagnetischen Feldes im Hochfrequenzbereich wird ein Plasma generiert, das sehr einheitlich bezüglich Intensität und Ausbreitung ist, und somit eine einheitliche Plasmabehandlung des Hohlkörpers gewährleistet.

Besonders bevorzugt liegt er Frequenzbereich im kHz- bis MHz-Bereich, insbesondere bevorzugt im Bereich von 1 kHz - 100 MHz. Dadurch wird ein besonders einheitliches Plasma generiert.

Insbesondere im Vergleich zu einem Mikrowellenplasma ist ein Hochfrequenzplasma energetisch günstiger generierbar. Dadurch sind reduzierte Verfahrenskosten möglich. Auch wird bei herkömmlichen Verfahren mit Mikrowellenplasmen größtenteils eine Vielzahl von Einzelkammern benötigt, um ein einheitliches Plasma im Inneren der Hohlkörper zu generieren. Erfindungsgemäß ist es jedoch gelungen, eine Plasmabehandlung für mehrere Flaschen gleichzeitig über eine einzige U-förmige Elektrode zu erzielen. Dies führt zu erheblich reduzierten Anlagenausmaßen und reduzierten Anlagenkosten. Weiter sind erfindungsgemäße Hochfrequenzplasmen wenig störanfällig, d.h. es ist im Vergleich zu Mikrowellenplasmen leichter, ein einheitliches Plasma zu erzeugen, d.h. es resultiert eine besonders gleichmäßige Plasmabehandlung.

Eine weitere Ausführungsform der Vorrichtung umfasst als Mittel zum Erzeugen des Plasmas eine oder mehrere rohrförmige Gegenelektroden, die sich während der Durchführung der Plasmabehandlung zumindest teilweise im Inneren der Hohlkörper befinden, und wobei die Gegenelektroden entlang ihrer Längsachse mehrere Öffnungen zum Einbringen von Gasen in den Hohlkörper aufweisen. Durch die rohrförmigen Gegenelektroden in Verbindung mit der U-förmigen Elektrode wird ein Plasma generiert, mit dem die Hohlkörper behandelt werden. Die rohrförmige Gegenelektrode weist entlang ihrer Längsachse mehrere Öffnungen auf. Dadurch können Prozessgase gleichmäßig im Hohlkörper umgesetzt werden, wodurch eine besondere hohe Einheitlichkeit des Plasmas resultiert. Weiter können an der Unterseite der rohrförmigen Gegenelektrode zusätzlich ein oder mehrere Öffnungen angebracht sein. Die Vielzahl, sowie die geeignete Verteilung der Öffnungen über die rohrförmige Elektrode hat den Vorteil, dass Prozessgase im Hohlkörper gleichmäßig und effektiv verteilt werden, und dadurch eine möglichst gleichmäßige Plasmabehandlung erfolgt.

Die Öffnungen können über die gesamte Länge der rohrförmigen Gegenelektrode erstrecken und / oder sich am unteren Ende der Elektrode befinden.

Insbesondere ist bevorzugt, dass die Öffnungen in der rohrförmigen Gegenelektrode so angeordnet sind, dass die Anzahl und/oder der Öffnungsdurchmesser im unteren Bereich der Elektrode, d.h. in der Nähe des Behälterbodens erhöht ist. Dadurch wird eine besonders einheitliche Plasmabehandlung gewährleistet. Der Durchmesser der Öffnungen liegt bevorzugt im mm Bereich, insbesondere im Bereich von 0,5 mm. Der Abstand der Öffnungen entlang der Längsache ist insbesondere im Bereich von ca. 8 - 25 mm. Weiter können entlang des Umfangs der rohrförmigen Gegenelektrode mehrere Öffnungen nebeneinander angebracht sein.

Der Außendurchmesser der rohrförmigen Gegenelektrode ist insbesondere im mm Bereich, bevorzugt bei ca. 5 bis 10 mm. Größere Durchmesser erschweren bzw. verhindern gegebenenfalls das Einführen der rohrförmigen Gegenelektrode in den Hohlkörper, sowie das Absaugen der Abgase durch die Mündung der Hohlkörper. Kleinere Durchmesser haben den Nachteil, dass die mechanische Stabilität der rohrförmigen Gegenelektrode ungenügend ist.

Die Länge der rohrförmigen Gegenelektrode ist an die Höhe des Hohlkörpers 4 angepasst, in der Art, dass bevorzugt zwischen dem Boden des Hohlkörpers 4 und dem unteren Ende der rohrförmigen Gegenelektrode maximal ca. 50 mm Abstand ist. Bei größeren Anständen ist die Einheitlichkeit der Plasmabehandlung nicht gewährleistet, da das elektromagnetische Feld nicht mehr einheitlich ist.

Die rohrförmige Gegenelektrode ist insbesondere so gestaltet, dass deren Länge variabel ist und an unterschiedliche Längen der Hohlkörper angepasst werden kann. Das ermöglicht eine variable, effektive und kostengünstige Ausgestaltung der Vorrichtung. Eine weitere Ausführungsform umfasst eine austauschbare rohrförmige Gegenelektrode. Dadurch kann leicht eine Anpassung an unterschiedliche Längen des Hohlkörpers erfolgen. Ein weiterer Vorteil ist die leichte Austauschbarkeit aufgrund Wartung oder Ähnlichem.

Die rohrförmige Gegenelektrode besteht im Wesentlichen aus einem elektrisch leitenden Material, insbesondere Kupfer oder Edelstahl, und ist über den Generator mit der U-förmigen Elektrode elektrisch leitend verbunden.

In einer weiteren Ausführungsform weist die rohrförmige Gegenelektrode in mindestens einem Bereich, bevorzugt an dem dem Hohlkörperboden zugewandten Ende, mindestens einen Magneten auf. Dadurch ist es möglich das elektromagnetische Feld, und somit die Plasmabehandlung gezielt zu beeinflussen. Insbesondere kann so ein einheitliches Plasma im gesamten Bereich des Hohlkörpers generiert werden, was eine möglichst effektive und einheitliche Plasmabehandlung gewährleistet. Weiter ist gezielt eine vermehrte Plasmabehandlung von Teilbereichen der Hohlkörper wie insbesondere dem Hohlkörperboden möglich. Insbesondere dienen die Magnete dazu, eine Beschichtung der rohrförmigen Gegenelektroden zu verhindern oder zumindest stark zu reduzieren.

Die Anzahl, die Art, sowie die Anordnung der Magnete ist nicht limitiert. Die Magnete können aus einem oder mehreren Einzelmagneten bestehen, und sind insbesondere kommerziell erhältliche, stabförmige Permanentmagnete, bevorzugt aus einer Cobalt/Samarium Legierung.

In einer alternativen Ausführungsform der Vorrichtung umfasst die U-förmige Elektrode mehrere Einzelsegmente. Diese Untergliederung in mehrere Einzelsegmente hat den Vorteil, dass die benötigte Leistung pro Einzelsegment reduziert wird, was einen besonders effektiven und ökonomischen Vorrichtungsaufbau gewährleistet. Weiter ist es dadurch möglich, eine unterschiedliche Plasmabehandlung in den einzelnen Segmenten durchzuführen, was eine besonders eine variable Plasmabehandlung bedingt.

In einer weiteren bevorzugten Ausführungsform der Vorrichtung umfasst die Unterdruckbehandlungskammer ein Mittel zur Kontrolle der Einheitlichkeit der Plasmabehandlung der Hohlkörper untereinander. Dieses Mittel ist insbesondere ein Analysegerät zur Aufzeichnung von spektroskopischen Parametern des Plasmas. Dadurch ist eine Kontrolle jedes einzelnen Hohlkörpers möglich.

Die oben beschriebenen Aufgaben werden weiter mit einem Verfahren nach Anspruch 7 erfindungsgemäß dadurch gelöst, dass die Hohlkörper während der Durchführung der Plasmabehandlung zumindest teilweise in den Innenbereich einer das elektromagnetische Feld generierenden U-förmigen Elektrode eintauchen, und zumindest zeitweise relativ zum elektromagnetischen Feld bewegt werden.

Eine bevorzugte Ausgestaltung des Verfahrens umfasst die folgenden Schritte: Einführen einer stabförmigen Gegenelektrode in das Innere der Hohlkörper; Einstellen eines geeigneten Druckes in den Hohlkörpern und in der Unterdruckbehandlungskammer; Zugabe eines oder mehrerer Prozessgase und Generieren eines elektromagnetischen Feldes; Generieren eines Plasmas im Inneren der Hohlkörper, in der Art dass sich die Hohlkörper während der Plasmabehandlung zumindest zeitweise im Innenbereich der U-förmigen Elektrode befinden, und die Hohlkörper relativ zur U-förmigen Elektrode bewegt werden. Durch einen solchen Verfahrensverlauf ist eine effektive kostengünstige Verfahrensführung möglich.

In einer bevorzugten Ausführungsform wird im Inneren der Hohlkörper ein erster reduzierter Druck (P2) eingestellt, und im Inneren der Unterdruckbehandlungskammer, aber auch außerhalb der Hohlkörper ein zweiter reduzierter Druck (P1) eingestellt, wobei der erste Druck (P2) geringer, bevorzugt mindestens 10 bis 2000 mal geringer als der zweite Druck (P1) ist. Dadurch wird gewährleistet, dass innerhalb des erfindungsgemäßen Verfahrens die Plasmabehandlung nur im Inneren der Hohlkörper stattfinden, was eine besonders effektive Plasmabehandlung gewährleistet.

Nach einer weiteren Ausgestaltung der Erfindung wird das Plasma durch ein elektromagnetisches Feld im Hochfrequenzbereich generiert. Dadurch ist eine besonders effektive und besonders einheitliche Plasmabehandlung der Hohlkörper möglich. Weiter ist das Erzeugen eines Plasmas durch ein elektromagnetisches Feld gemäß dieser Ausführungsform sehr kostengünstig und effektiv generierbar.

In einer weiteren Ausführungsform des Verfahrens wird im Verlauf der Plasmabehandlung ein einzelner Hohlkörper mit unterschiedlichen Plasmen behandelt, die aufgrund unterschiedlicher elektromagnetischer Felder bezüglich Feldstärke und/oder Frequenz generiert werden. Dadurch sind eine besonders effektive Plasmabehandlung des Hohlkörpers möglich, und ein besonders flexibler Verfahrensaufbau realisierbar.

Eine bevorzugte Ausgestaltung ist dadurch gekennzeichnet, dass das elektromagnetische Feld durch einen oder mehrere Magnete im Inneren der rohrförmigen Gegenelektrode beeinflusst wird. Ionen und Elektronen können durch Magnete ablenkt bzw. kontrolliert werden. Dadurch kann über eine geeignete Anordnung der Magnete innerhalb der rohrförmigen Elektrode der Elektronen- und Ionenstrom, und damit auch die Plasmagüte innerhalb der Hohlkörper so eingestellt werden, dass eine möglichst gleichmäßige bzw. bevorzugte Plasmabehandlung der Hohlkörper möglich wird. Insbesondere im Fall einer Plasmabeschichtung wird dadurch ein Beschichten der Elektrode verhindert oder zumindest stark reduziert.

Nach einer bevorzugten Ausgestaltung des Verfahrens erfolgt eine Zugabe von Prozessgasen in den Hohlkörper über mehrere Öffnungen an der rohrförmigen Gegenelektrode, die sich bevorzugt entlang der Längsachse, entlang des Umfangs, sowie insbesondere am unteren Ende der stabförmigen Gegenelektrode befinden. Dadurch kann der Eintrag an Prozessgasen so eingestellt werden, dass sich eine gleichmäßige Plasmabehandlung im gesamten Hohlkörper ergibt. Dadurch ist ein effektiver, schneller und kostengünstiger Verfahrensverlauf möglich.

Bedingt durch den Plasmaprozess werden die rohrförmigen Elektroden während der Durchführung der Plasmabehandlung erhitzt. Dies hat den Vorteil, dass insbesondere bei einer Plasmabeschichtung keine Schichtabscheidung an der Oberfläche der rohrförmigen Gegenelektrode stattfindet, bzw. die Schichtabscheidung deutlich verringert wird. Die Erhitzung der rohrförmigen Gegenelektrode wird im Falle der Verwendung von Hochfrequenzenergie ohne aktive Heizelemente oder ähnliches erzielt, da hier aufgrund elektrischer Reibungsverluste eine Erwärmung der Elektroden eintritt. Dies ist insbesondere als Vorteil zu mikrowellengenerierten Plasmen anzusehen, da dort eine Erwärmung der rohrförmigen Gegenelektrode weniger stark auftritt. Die Temperatur der rohrförmigen Gegenelektrode während der Durchführung des Verfahrens liegen insbesondere im Bereich maximal ca. 100 °C. Der Vorteil einer solchen Erhitzung liegt insbesondere darin, dass die Öffnungen in der rohrförmigen Elektrode bei einer Plasmabeschichtung nicht, bzw. weniger stark beschichtet werden, und so erfindungsgemäß ein gleichmäßiger Gaseintrag über eine lange Zeitdauer möglich ist. Somit ist eine effektive und kostengünstige Verfahrensführung möglich, da eine Wartung d.h. ein Auswechseln der stabförmigen Elektroden nicht oder nur in verringertem Umfang nötig ist.

Insbesondere umfasst die Erfindung ein Verfahren, bei der die Plasmabehandlung ein PECVD-Prozess (plasma enhanced chemical vapor deposition) zur Innenbeschichtung der Hohlkörper mit insbesondere einer SiOx-Schicht ist. Durch eine solche Plasmabehandlung kann in effektiver und kostengünstiger Weise die Gaspermeabilität von Hohlkörpern, insbesondere von PET-Flaschen, reduziert werden, so dass die Permeabilität gegenüber Gasen, wie z.B. Sauerstoff und/oder Kohlendioxid, erheblich reduziert wird.

Die Schichtdicken sind insbesondere im Nanometerbereich, bevorzugt im Bereich von 1 bis 100 nm, besonders bevorzugt im Bereich von 10 bis 30 nm. Bei dünneren Schichten ist nicht gewährleistet, dass eine einheitliche Schicht die Oberfläche bedeckt. Dickere Schichten haben den Nachteil, dass die Schicht spröde und rissig wird, und so, insbesondere bei Verformung des beschichteten Hohlkörpers, die Schichteigenschaften, insbesondere die Barriereeigenschaften der Schicht verringern.

Insbesondere dient das Verfahren zur Ausbildung einer einheitlichen Schichtdicke. Die minimale bzw. maximale Schichtdicke liegt bevorzugt bei 50% bzw. 150% der mittleren Schichtdicke. Dadurch wird eine besonders effektive Barriere gegenüber Gasen erzielt. Ist die Schichtdicke uneinheitlicher, ist die Barrierewirkung nicht ausreichend und/oder nur eine uneffektive Verfahrensführung möglich, da die Gesamtschichtdicken erhöht werden müssen.

In einer weiteren bevorzugten Ausführungsform wird das Verfahren bezüglich der Einheitlichkeit der Plasmasbehandlung jedes einzelnen Hohlkörpers kontrolliert. Dabei wird insbesondere das Plasma im Inneren der Hohlkörper spektroskopisch untersucht, indem relevante spektroskopische Parameter durch ein Analyseinstrument aufgezeichnet werden. In Behältern mit ungenügenden Parametern wird erfindungsgemäß ein nachträgliches Aussondern der betroffenen Hohlkörper ermöglicht. Insbesondere ist auch möglich, über einen Regelkreis Verfahrensparameter anzupassen, so das eine einheitliche Verfahrensführung möglich ist.

Die Erfindung und ihre Vorteile werden anhand der in den nachfolgenden Zeichnungen dargestellten Ausführungsbeispiele weiter erklärt. Dabei zeigen:
- Fig. 1: eine schematische Schnittzeichnung einer erfindungsgemäßen Vorrichtung;
- Fig. 2: eine vergrößerte Schnittdarstellung der Vorrichtung mit Hohlkörper;
- Fig. 3: eine schematische Schnittzeichnung der rohrförmigen Gegenelektrode;
- Fig. 4: eine schematische Schnittdraufsicht einer erfindungsgemäßen Vorrichtung;
- Fig. 5: eine schematische Schnittzeichnung in der Draufsicht einer bevorzugten Ausführungsform der erfindungsgemäßen Vorrichtung, bei der die U-förmige Elektrode segmentartig angeordnet ist;
- Fig. 6: eine schematische Schnittzeichnung eines plasmabeschichteten Hohlkörpers gemäß einer bevorzugten Ausführungsform der Erfindung.
- Fig. 7: eine schematische Darstellung einer bevorzugten Ausführungsform der Vorrichtung als Rundläufer;

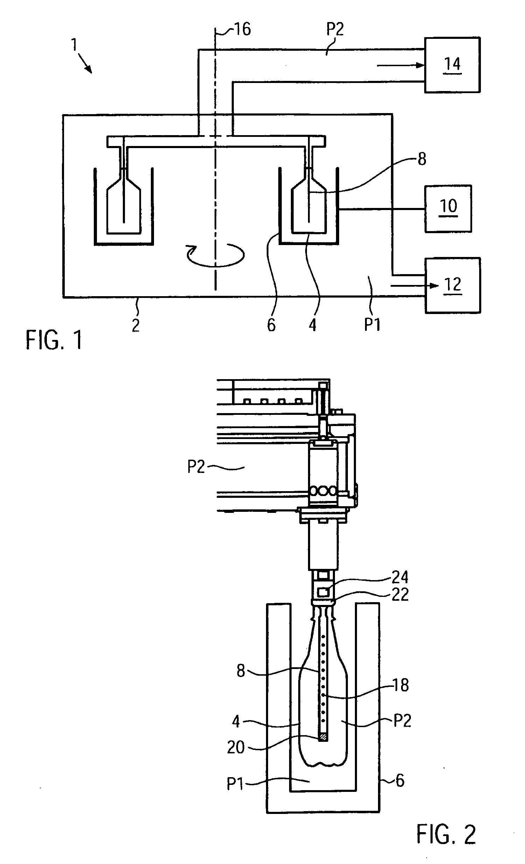

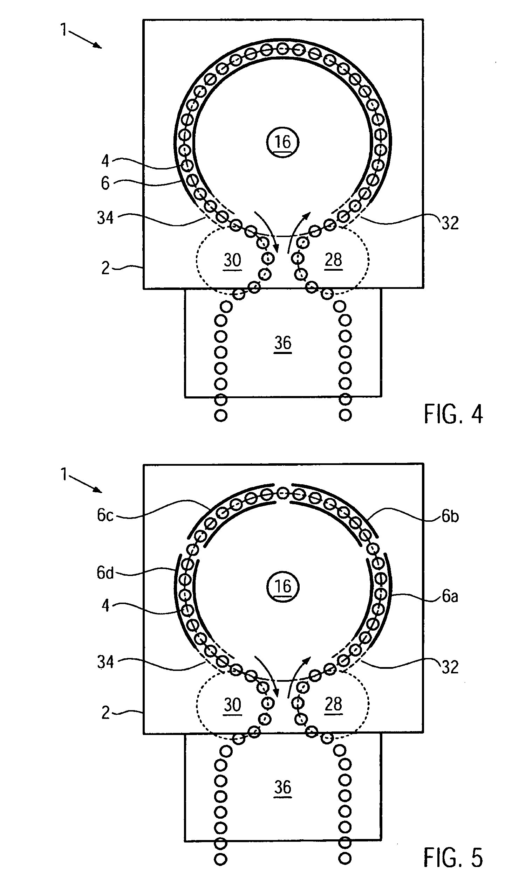

Fig. 1 zeigt schematisch eine Vorrichtung 1 mit einer Unterdruckbehandlungskammer 2 und einer U-förmigen Elektrode 6 zur Aufnahme der zu behandelnden Hohlkörper 4. Wie aus Fig. 4 ersichtlich ist, erstreckt sich die U-förmige Elektrode 6 über einen Winkel von ca. 220°. Die U-förmige Elektrode 6 ist horizontal auf einer Kreisbahn in der Unterdruckbehandlungskammer 2 angeordnet. In der Unterdruckbehandlungskammer 2 befinden sich mehrere rohrförmige Gegenelektroden 8, die während der Plasmabehandlung ins Innere je eines Hohlkörpers 4 ragen. Weiter befindet sich ein Mittel zur Beförderung der Hohlkörper 16 in der Gestalt eines Rotors in der Unterdruckbehandlungskammer 2, mit dem die Hohlkörper 4 relativ zur U-förmigen Elektrode 6 bewegt werden können, in der Art, dass die Hohlkörper 4 in einer Kreisbahn durch das Innere der U-förmigen Elektrode 6 bewegt werden. Die Vorrichtung 1 umfasst zwei separate Pumpensysteme, bzw. Absaugvorrichtungen12 und 14. Pumpensystem 12 generiert einen Unterdruck (P1) in der Unterdruckbehandlungskammer 2, das Pumpensystem 14 generiert einen Unterdruck (P2) in den Hohlkörpern 4. Der Druck (P2) ist mindestens 10 bis 2000 mal geringer als der Druck (P1) ist. Insbesondere ist der Druck (P2) ca. 2 Pa, und der Druck (P1) ca. 3000 Pa. Während der Plasmabehandlung erfolgt kontinuierlich eine Absaugung aus dem Hohlkörper 4 über Pumpensystem 14.

Die U-förmige Elektrode 6 ist mit dem Generator 10 elektrisch verbunden und gegenüber dem Gehäuse der Behandlungskammer 2 elektrisch isoliert. Der Generator 10 ist außerhalb der Unterdruckbehandlungskammer angeordnet und sein Gehäuse geerdet. Der Generator kann eine elektrische Wechselspannung zwischen der U-förmigen Elektrode 6 und der ebenfalls geerdeten rohrförmigen Gegenelektrode 8 erzeugen. Dadurch wird im Inneren der U-förmigen Elektrode 6 ein elektromagnetisches Feld im Hochfrequenzbereich generiert. Das elektromagnetische Feld ist im kHz- bis MHz-Bereich, insbesondere im Bereich von 1 kHz bis 100 MHz. Die Generatorleistung beträgt zum Beispiel 20 KW. Der Generator 10 kann aus mehreren Einzelgeneratoren bestehen, zum Beispiel aus vier Generatoren je 5 KW.

In der Vorrichtung können herkömmliche Halte- bzw. Transportsysteme zur Halterung bzw. zum Transport der Hohlkörper 4 verwendet werden (nicht gezeigt). Die Greifer sind bevorzugt elektrisch gegenüber der Gehäusemasse isoliert, um ein Plasma zwischen den Greifern und der U-förmigen Elektrode zu verhindern.

Fig. 2 zeigt einen vergrößerten Ausschnitt der Vorrichtung 1, bei dem eine U-förmige Elektrode 6 dargestellt ist, in die ein Hohlkörper 4 eintaucht. Innerhalb des Hohlkörpers 4 befindet sich eine rohrförmige Gegenelektrode 8. Der Hohlkörper 4 wird vom Innenraum der Unterdruckbehandlungskammer 2 gasdicht über eine Dichtvorrichtung 22 abgeschlossen. Die Dichtvorrichtung 22 ist mit einem Ventil 24 kombiniert, das während der Plasmabehandlung geöffnet wird, so dass eine Absaugung der Hohlkörper 4 durch das Pumpensystem 14 auf den Druck P2 erfolgen kann.

In einer alternativen Ausführungsform ist der Ventilanschluss, d.h. das Ventil 24 und die Dichtvorrichtung 22, drehbar, so dass die Behälter 4 während der Beschichtung gedreht werden. Dadurch ist eine besonders gleichmäßige Plasmabehandlung möglich.

Die Seitenwände der U-förmigen Elektrode 6 sind parallel zur rohrförmigen Gegenelektrode 8 angeordnet. Der Abstand der rohrförmigen Gegenelektrode 8 zu den Seitenwänden der U-förmigen Elektrode 6 ist im Wesentlichen gleich. Dadurch ist ein einheitliches elektromagnetisches Feld im Inneren der U-förmigen Elektrode 6 generierbar, so dass eine möglicht einheitliche Plasmabehandlung der Hohlkörper 4 erfolgen kann.

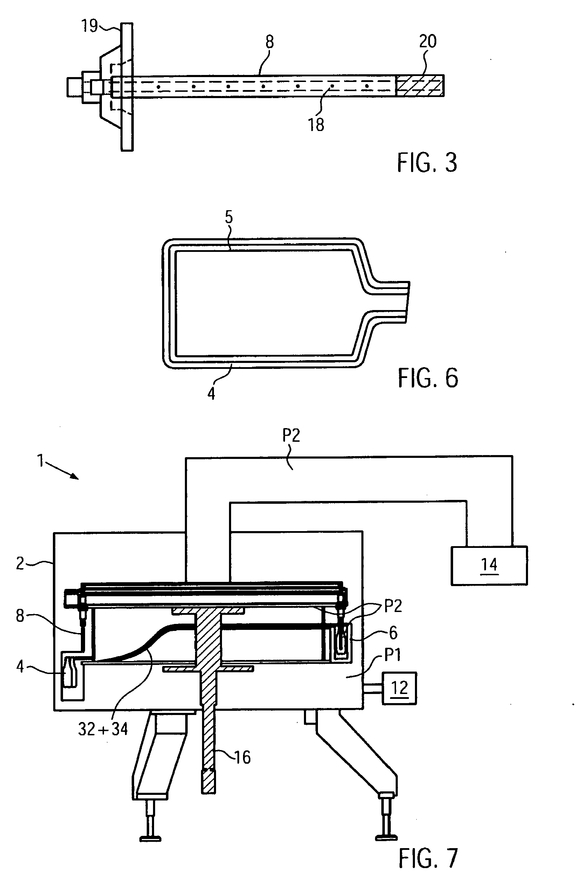

Figur 3 zeigt detailliert die Ausgestaltung der rohrförmigen Gegenelektrode 8. Der Außendurchmesser der rohrförmigen Gegenelektrode 8 liegt bei ca. 10 mm. Die rohrförmige Gegenelektrode 8 ist ein Hohlrohr und weist eine Vielzahl von Öffnungen 18 auf, durch die Prozessgase ins Innere des Hohlkörpers 4 eingeleitet werden. Der Durchmesser der Öffnungen 18 liegt bei ca. 0,3 mm. Entlang der Längsachse der rohrförmigen Gegenelektrode 8 sind jeweils sieben Öffnungen 18 angebracht, entlang der Querachse jeweils vier auf einer Höhe in einem Winkel von 90°, so dass die rohrförmige Gegenelektrode 8 insgesamt 28 seitliche Öffnungen 18 aufweist. Der Abstand der Öffnungen 18 entlang der Längsache liegt zwischen ca. 8-25 mm. Der Anstand der Öffnungen 18 entlang der Längsachse ist unregelmäßig, in der Art dass der Abstand zwischen den Öffnungen in Richtung des Elektrodenendes bzw. des Behälterbodens abnimmt. Zusätzlich sind am unteren Ende der rohrförmigen Elektrode 8 ein oder mehrere weitere Öffnungen 18 angebracht. Durch diese Verteilung der Öffnungen 18 kann der Eintrag an Prozessgasen so eingestellt werden, dass sich eine gleichmäßige Plasmabehandlung im gesamten Hohlkörper ergibt, und ein effektiver, schneller und kostengünstiger Verfahrensverlauf möglich ist.

Die Länge der rohrförmigen Gegenelektrode 8 ist an die Höhe des Hohlkörpers 4 angepasst. Der Abstand zwischen dem Boden des Hohlkörpers 4 und dem unteren Ende der rohrförmigen Gegenelektrode 8 beträgt maximal ca. 50 mm. Bei größeren Anständen ist die Einheitlichkeit der Plasmabehandlung nicht gewährleistet, da das elektromagnetische Feld nicht mehr einheitlich ist.

Die Länge der rohrförmigen Gegenelektrode 8 ist variabel einstellbar, indem die rohrförmige Gegenelektrode 8 relativ zur Dichtvorrichtung 22 verschoben wird. Das ermöglicht eine variable, effektive und kostengünstige Ausgestaltung der Vorrichtung 1, da die Länge der rohrförmigen Gegenelektrode 8 leicht und schnell an unterschiedliche Längen des Hohlkörpers 4 angepasst werden kann.

Weiter umfasst die rohrförmigen Gegenelektrode 8 eine Halterungseinheit 19, mit der eine Befestigung an der Dichtvorrichtung 22 erfolgt. Die Halterungseinheit 19 ist so ausgestaltet, dass ein schnelles und effektives Wechseln der rohrförmigen Gegenelektrode 8 über eine Schraub- oder Steckeinheit möglich ist. Über den Austausch der rohrförmigen Gegenelektroden 8 kann eine Anpassung an unterschiedliche Längen des Hohlkörpers 4 erfolgen. Ein weiterer Vorteil ist die schnelle und einfache Austauschbarkeit aus Wartungsgründen.

Die rohrförmige Gegenelektrode 8 besteht im wesentlichen aus einem elektrisch leitenden Material, insbesondere Kupfer oder Edelstahl, und ist über den Generator 10 mit der U-förmigen Elektrode 6 elektrisch leitend verbunden.

Im Inneren der rohrförmigen Gegenelektrode 8 befindet sich ein Stabmagnet (nicht gezeigt) aus einer Cobalt/Samarium Legierung. Die rohrförmige Gegenelektrode 8 weist eine abnehmbare Hülse 20 auf, über der Magnet ausgetauscht werden kann. Der Magnet erstreckt sich in seiner Länge vom Ende der rohrförmigen Elektrode bis zu dem Bereich in dem der Hohlkörperdurchmesser kleiner wird. Durch den Magnet wird das Plasma derart verändert, dass die Elektrode nicht beschichtet wird, und so eine besonders effektive Verfahrensführung möglich ist. Weiter ist dadurch eine einheitliche Plasmabehandlung gewährleistet.

Fig. 4 stellt eine Ausgestaltung der Vorrichtung 1 in der Draufsicht dar. Die Vorrichtung 1 umfasst eine Schleusenvorrichtung 36 zur Einschleusung der Hohlkörper 4 in die Unterdruckbehandlungskammer 2. Weiter beinhaltet die Vorrichtung 1 einen Einlaufstern 30, sowie einen Auslaufstern 28, zur Übergabe der Hohlkörper 4 auf einen Rotor 16. Rotor 16 dient dazu, die Hohlkörper 4 während der Durchführung der Plasmabehandlung relativ zur U-förmigen Elektrode 6 zu bewegen. Über die Hubkurven 34 und 32 werden die Hohlkörper 4 vom Höheniveau des Einlaufsterns 30 und Auslaufsterns 28 auf das Niveau der U-förmigen Elektrode 6 gebracht. In Fig. 7 ist die Vorrichtung 1 nochmals als Schnittzeichnung dargestellt. Die Hohlkörper 4 sind mit dem Rotor 16 über eine Halterungsvorrichtung (nicht gezeigt) verbunden. Dies erfolgt über ein sogenanntes Neck-Handling, d.h. die Halterung erfolgt über den Hohlkörperhals. Solche Halterungs/Handlingssysteme sind aus dem Stand der Technik bekannt.

Fig. 5 zeigt eine alternative Ausführungsform der Vorrichtung 1, bei der die U-förmige Elektrode 6 segmentartig unterteilt ist, so dass sich vier Segmente 6a-d ausbilden. Das Vorhandensein dieser segmentartig untergliederten U-förmigen Elektrode 6 hat den Vorteil, dass die Leistung, die erforderlich ist, um an der U-förmigen Elektrode 6 ein geeignetes elektromagnetisches Feld gegenüber den rohrförmigen Gegenelektroden 8 zu generieren, verringert wird, so dass ein besonders effektiver und kostengünstiger Verfahrensverlauf möglich ist.

In der Vorrichtung 1 kann ein Verfahren wie folgt durchgeführt werden:

Über eine Schleusenvorrichtung 36 werden eine Vielzahl von Hohlkörpern 4 kontinuierlich in die Unterdruckbehandlungskammer 2 eingeführt, in der über die Absaugvorrichtung 12 ein Unterdruck (P1) erzeugt wird. Über einen Einlaufstern 30 erfolgt die Übergabe der Hohlkörper 4 auf einen Rotor 16. Die Hohlkörper 4 werden über die Rotation des Rotors 16 kreisförmig bewegt. Über eine Hubkurve 34 wird durch Fortschreiten des Rotationsvorgangs je ein Hohlkörper über eine Gegenelektrode 8 geführt, so dass die rohrförmige Gegenelektrode 8 in das Innere des Hohlkörpers 4 zeigt. Gleichzeitig erfolgt durch die Hub/Rotationsbewegung ein zumindest teilweises Eintauchen der Hohlkörper 4 in den Innenbereich einer U-förmigen Elektrode 6. Durch das Anheben werden die Hohlkörper 4 gegen eine Dichtvorrichtung 22 gepresst, und so ein gasdichter Verschluss des Hohlkörpersinnenbereichs bezüglich der Unterdruckbehandlungskammer 2 erzielt. Dadurch wird ein Ventil 24 geöffnet, so dass über die Absaugvorrichtung 12 im Inneren der Hohlkörper 4 ein Unterdruck (P2) generiert wird und kontinuierlich eine Absaugung erfolgt. Anschließend werden Prozessgase über die Öffnungen 18 der rohrförmigen Gegenelektrode 8 ins Innere der Hohlkörper 4 geleitet. Der Generator 10, der elektrisch leitend mit der U-förmigen Elektrode 6, verbunden ist, generiert nun innerhalb des Innenbereichs der U-förmigen Elektrode 6 ein elektromagnetisches Feld gegenüber den geerdeten rohrförmigen Gegenelektroden 8. Durch das Fortschreiten der Rotation des Rotors 16 werden die Hohlkörper 4 durch dieses elektromagnetische Feld bewegt und ein Plasma im Inneren der Hohlkörper generiert. D.h. die Plasmabehandlung der Hohlkörper 4 erfolgt, während sich die Hohlkörper 4 im Innenbereich der U-förmigen Elektrode befinden und relativ zur U-förmigen Elektrode bewegt werden. Nach der Plasmabehandlung erfolgt über eine Hubkurve 32 eine Abwärtsbewegung der Hohlkörper 4. Dadurch wird die Dichtvorrichtung 22 gelöst das Ventil 24 geschlossen, so dass sich im Inneren der Hohlkörper der Druck (P1), der in der Unterdruckbehandlungskammer 2 vorherrscht, einstellt. Durch das Fortschreiten der Rotationsbewegung treten die Hohlkörper aus dem Innenbereich der U-förmigen Elektrode 6 aus, und die rohrförmigen Gegenelektroden 8 werden durch die Abwärtsbewegung aus dem Inneren der Hohlkörper entfernt. Dadurch erlischt das Plasma im Inneren der behandelten Hohlkörper 4. Anschließend erfolgt eine Übergabe der Hohlkörper 4 vom Rotor 16 auf einen Auslaufstern 28, sowie das Ausschleusen aus der Unterdruckbehandlungskammer 2.

Durch das Verfahren ist es möglich, mit einer einzigen U-förmigen Elektrode 6 ein Plasma in einer Vielzahl von Hohlkörpern 4 zu generieren. Durch das Vorhandensein des elektromagnetischen Feldes über den gesamten Innenbereich der U-förmigen Elektrode 6 wird auch bei der Relativbewegung der Hohlkörper 4 eine Einwirkung des Plasmas auf jeden einzelnen Hohlkörper über einen längeren Zeitraum gewährleistet. Dadurch sind keine Einzelkammern erforderlich, da sich alle in der Vorrichtung befindenden Hohlkörper in der gleichen U-förmigen Elektrode befinden. Somit ist eine besonders effektive und kostengünstige Verfahrensführung gegeben, da auf das Einbringen der Hohlkörper 4 in eine Vielzahl von Einzelkammern verzichtet werden kann.

Der Druck wird so eingestellt, dass Druck (P2) im Inneren der Hohlkörper 4 mindestens 10 bis 2000 mal geringer als der Druck in der Vakuumbehandlungskammer 2 (P1) ist. Dadurch wird gewährleistet, dass die Plasmabehandlung ausschließlich im Inneren der Hohlkörper 4 stattfindet. Dies hat den Vorteil, dass ausschließlich das Innere der Hohlkörper behandelt wird. D.h. der Ort der Plasmabehandlung kann gezielt gesteuert werden, womit ein effektiver, energetisch günstiger Verfahrensverlauf möglich ist.

Die Frequenz des elektromagnetischen Feldes liegt im Hochfrequenzbereich, bevorzugt im kHz- bis MHz-Bereich, insbesondere im Bereich von 1 kHz bis 100 MHz. Dadurch ist eine besonders effektive und kostengünstige Verfahrensführung möglich.

In einer alternativen Ausführungsform des Verfahrens gemäß Figur 5 wird im Verlauf der Plasmabehandlung ein Hohlkörper mit unterschiedlichen Plasmen behandelt. Dies wird dadurch erreicht, dass die U-förmige Elektrode 6a-d segmentartig aufgebaut ist, und so unterschiedliche elektromagnetischer Felder bezüglich Feldstärke und/oder Frequenz generiert werden. Dies hat zum Vorteil, dass im Verlauf der Plasmabehandlung eines Hohlkörpers innerhalb der U-förmigen Elektrode 6 der Hohlkörper 4 durch die Relativbewegung zur U-förmigen Elektrode 6 verschiedene elektromagnetische Felder durchläuft, und dadurch die Plasmabehandlung unterschiedlich ist.

Die Zugabe der Prozessgase in den Hohlkörper erfolgt über die Öffnungen 18, die sich entlang der Längsachse der rohrförmigen Gegenelektrode 8 finden. Die gleichzeitige Zugabe der Prozessgase über eine Vielzahl von Öffnungen 18 hat den Vorteil, dass die Prozessgase derart in den Hohlkörpern 4 zugegeben werden, dass eine gleichmäßige und besonders effektive Plasmabehandlung möglich ist. Insbesondere im Falle einer Plasmabeschichtung kann dadurch eine möglichst einheitliche Schichtdicke erzielt werden. Dadurch ist ein effektiver, schneller und kostengünstiger Verfahrensverlauf möglich.

Die rohrförmigen Gegenelektroden 8 werden während der Durchführung der Plasmabehandlung erhitzt. Dies hat den Vorteil, dass insbesondere bei einer Plasmabeschichtung keine Schichtabscheidung an der Oberfläche der rohrförmigen Gegenelektrode 8 stattfindet, bzw. die Schichtabscheidung deutlich verringert wird. Die Erhitzung der rohrförmigen Gegenelektrode 8 wird aufgrund der Verwendung von Hochfrequenzenergie ohne aktive Heizelemente oder ähnliches erzielt, da hier aufgrund des Plasmabetriebes eine Erwärmung der Elektroden eintritt. Die Temperatur der rohrförmigen Gegenelektrode 8 während der Durchführung des Verfahrens liegen im Bereich von maximal ca. 100°C. Der Vorteil einer solchen Erhitzung liegt insbesondere darin, dass die Öffnungen 18 bei einer Plasmabeschichtung nicht, bzw. weniger stark beschichtet werden und so erfindungsgemäß ein gleichmäßiger Gaseintrag über eine lange Zeitdauer möglich ist. Somit ist eine effektive und kostengünstige Verfahrensführung möglich, da eine Wartung d.h. ein Auswechseln der stabförmigen Elektrode 8 nicht oder nur in verringertem Umfang nötig ist.

Die Vorrichtung 1 dient zur Durchführung eines Plasmabehandlungsverfahrens von Hohlkörpern 4 und insbesondere zur Durchführung eines PECVD Prozesses zur Innenbeschichtung mit einer SiOx Schicht 5. Alternativ kann auch eine DLC-Schicht abgeschieden werden. Ein beschichteter Hohlkörper ist in Fig. 6 dargestellt.

## Patentansprüche

1. Vorrichtung zur Plasmabehandlung von Hohlkörpern (4), umfassend eine Unterdruckbehandlungskammer (2) und Mittel zum Erzeugen des Plasmas, **dadurch gekennzeichnet, dass** die Mittel zum Erzeugen des Plasmas eine im Querschnitt im wesentlichen U-förmige Elektrode (6) umfassen, die in der Unterdruckbehandlungskammer (2) angeordnet ist, wobei die Vorrichtung so eingerichtet ist, dass die Hohlkörper (4) während der Durchführung der Plasmabehandlung zumindest teilweise in die U-förmige Elektrode (6) eintauchen, und zumindest zeitweise relativ zur U-förmigen Elektrode (6) bewegt werden.

2. Vorrichtung nach Anspruch 1, **dadurch gekennzeichnet, dass** die Vorrichtung mindestens zwei separate Absaugvorrichtungen umfasst, wobei eine Absaugvorrichtung (14) im Inneren der Hohlkörper (4) einen ersten reduzierten Druck (P2) generiert, und eine separate Absaugvorrichtung (12) im Inneren der Unterdruckbehandlungskammer (2), aber außerhalb der Hohlkörper (4) einen zweiten reduzierten Druck (P1) generiert, und wobei die Absaugvorrichtungen (12, 14) so eingerichtet sind, dass der erste reduzierte Druck (P2) geringer, bevorzugt mindestens 10 bis 2000 mal geringer, als der zweite reduzierte Druck (P1) ist.

3. Vorrichtung nach einem der Ansprüche 1 bis 2, **dadurch gekennzeichnet, dass** die Mittel zum Erzeugen des Plasmas einen Generator (10) zum Erzeugen eines elektromagnetischen Feldes im Hochfrequenzbereich, bevorzugt im KHz bis MHz Bereich, umfasst.

4. Vorrichtung nach einem der Ansprüche 1 bis 3, **dadurch gekennzeichnet, dass** die Mittel zum Erzeugen des Plasmas eine oder mehrere rohrförmige Gegenelektroden (8) umfassen, die sich während der Durchführung der Plasmabehandlung zumindest zeitweise im Inneren der Hohlkörper (4) befinden, und wobei die rohrförmigen Gegenelektroden (8) entlang ihrer Längsachse mehrere Öffnungen (18) zum Einbringen von Gasen in den Hohlkörper (4) aufweisen.

5. Vorrichtung nach Anspruch 4, **dadurch gekennzeichnet, dass** die rohrförmige Gegenelektrode (8) in mindestens einem Bereich, bevorzugt am Hohlkörperboden zugewandtem Ende, mindestens einen Magnet umfasst.

6. Vorrichtung nach einem der Ansprüche 1 bis 5, **dadurch gekennzeichnet, dass** die U-förmige Elektrode (6) mehrere Einzelsegmente (6a-d) umfasst.

7. Verfahren zur Plasmabehandlung von Hohlkörpern (4), bei dem die Hohlkörper (4) in eine Unterdruckbehandlungskammer (2) verbracht werden, in der die Plasmabehandlung erfolgt, und das Plasma durch ein elektromagnetisches Feld generiert wird, **dadurch gekennzeichnet, dass** die Hohlkörper (4) während der Durchführung der Plasmabehandlung zumindest teilweise in den Innenbereich einer das elektromagnetische Feld generierenden U-förmigen Elektrode (6) eintauchen, und zumindest zeitweise relativ zum elektromagnetischen Feld bewegt werden.

8. Verfahren nach Anspruch 7, umfassend die folgenden Schritte:
- Einführen einer rohrförmigen Gegenelektrode (8) in das Innere der Hohlkörper (4);
- Einstellen eines geeigneten Druckes in den Hohlkörpern (4) und in der Unterdruckbehandlungskammer (2);
- Zugabe eines oder mehrerer Prozessgase und Generieren eines elektromagnetischen Feldes,
- Generieren eines Plasmas im Inneren der Hohlkörper (4), in der Art dass sich die Hohlkörper (4) während der Plasmabehandlung zumindest zeitweise im Innenbereich der U-förmigen Elektrode (6) befinden, und die Hohlkörper (4) relativ zur U-förmigen Elektrode (6) bewegt werden.

9. Verfahren nach einem der Ansprüche 7 bis 8, **dadurch gekennzeichnet, dass** im Inneren der Hohlkörper (4) ein erster reduzierter Druck (P2) eingestellt wird, und im Inneren der Unterdruckbehandlungskammer (2), aber außerhalb der Hohlkörper ein zweiter reduzierter Druck (P1) eingestellt wird, wobei der erste reduzierte Druck (P2) geringer, bevorzugt mindestens 10 bis 2000 mal geringer, als der zweite reduzierte Druck (P1) ist.

10. Verfahren nach einem der Ansprüche 7 bis 9, **dadurch gekennzeichnet, dass** das Plasma durch ein elektromagnetisches Feld im Hochfrequenzbereich, bevorzugt im KHz bis MHz Bereich, generiert wird.

11. Verfahren nach einem der Ansprüche 7 bis 10, **dadurch gekennzeichnet, dass** im Verlauf der Plasmabehandlung ein Hohlkörper (4) mit unterschiedlichen Plasmen behandelt wird, die aufgrund unterschiedlicher elektromagnetischer Felder bezüglich Feldstärke und/oder Frequenz generiert werden.

12. Verfahren nach einem der Ansprüche 7 bis 11, **dadurch gekennzeichnet, dass** das elektromagnetische Feld durch einen oder mehrere Magnete in der rohrförmigen Gegenelektrode (8) beeinflusst wird.

13. Verfahren nach einem der Ansprüche 7 bis 12, **dadurch gekennzeichnet, dass** die Zugabe des Prozessgases oder der Prozessgase in den Hohlkörper (4) über eine oder mehrere Öffnungen (18) erfolgt, die sich entlang der Längsachse der rohrförmigen Gegenelektrode (8) befinden.

14. Verfahren nach einem der Ansprüche 7 bis 13, **dadurch gekennzeichnet, dass** die rohrförmigen Gegenelektroden (8) während der Durchführung der Plasmabehandlung erhitzt werden.

15. Verfahren nach einem der Ansprüche 7 bis 14, **dadurch gekennzeichnet, dass** die Plasmabehandlung ein PECVD Prozess zur Innenbeschichtung der Hohlkörper, mit insbesondere einer SiOx Schicht, ist.

## Claims

1. An apparatus for the plasma treatment of hollow bodies (4), comprising a vacuum treatment chamber (2) and means for generating the plasma, **characterized in that** the means for generating the plasma comprise an electrode (6) of a substantially U-shaped cross-section, which is arranged in the vacuum treatment chamber (2), the apparatus being configured such that the hollow bodies (4) immerse at least in part into the U-shaped electrode (6) when the plasma treatment is carried out, and are moved at least temporarily relative to the U-shaped electrode (6).

2. The apparatus according to claim 1, **characterized in that** the apparatus comprises at least two separate suction devices, one suction device (14) generating a first reduced pressure (P2) in the interior of the hollow bodies (4), and a separate suction device (12) generating a second reduced pressure (P1) in the interior of the vacuum treatment chamber (2), but outside the hollow bodies (4), wherein the suction devices (12, 14) are configured such that the first reduced pressure (P2) is smaller, preferably at least 10 to 2000 times smaller, than the second reduced pressure (P1).

3. The apparatus according to any one of claims 1 to 2, **characterized in that** the means for generating the plasma comprises a generator (10) for generating an electromagnetic field in the high-frequency range, preferably in the kHz and MHz range.

4. The apparatus according to any one of claims 1 to 3, **characterized in that** the means for generating the plasma comprise one or more tubular counter electrodes (8) that are positioned at least temporarily in the interior of the hollow bodies (4) when the plasma treatment is carried out, and the tubular counter electrodes (8) along their longitudinal axis comprising a plurality of openings (18) for introducing gases into the hollow bodies (4).

5. The apparatus according to claim 4, **characterized in that** the tubular counter electrode (8) comprises at least one magnet in at least one portion, preferably at the end facing the hollow body bottom.

6. The apparatus according to any one of claims 1 to 5, **characterized in that** the U-shaped electrode (6) comprises a plurality of individual segments (6a-d).

7. A method for the plasma treatment of hollow bodies (4), wherein the hollow bodies (4) are moved into a vacuum treatment chamber (2) in which the plasma treatment is carried out and the plasma is generated by an electromagnetic field, **characterized in that** the hollow bodies (4) immerse at least in part into the inner portion of a U-shaped electrode (6) generating the electromagnetic field, when the plasma treatment is carried out, and are moved at least temporarily relative to the electromagnetic field.

8. The method according to claim 7, comprising the following steps:
- introducing a tubular counter electrode (8) into the interior of the hollow bodies (4);
- setting an appropriate pressure in the hollow bodies (4) and in the vacuum treatment chamber (2);
- adding one or more process gases and generating an electromagnetic field;
- generating a plasma in the interior of the hollow bodies (4) in such fashion that the hollow bodies (4) are positioned during plasma treatment at least temporarily in the inner portion of the U-shaped electrode (6), and the hollow bodies (4) are moved relative to the U-shaped electrode (6).

9. The method according to any one of claims 7 to 8, **characterized in that** a first reduced pressure (P2) is set in the interior of the hollow bodies (4) and a second reduced pressure (P1) is set in the interior of the vacuum treatment chamber (2), but outside the hollow bodies, with the first reduced pressure (P2) being smaller, preferably at least 10 to 2000 times, than the second reduced pressure (P1).

10. The method according to any one of claims 7 to 9, **characterized in that** the plasma is generated by an electromagnetic field in the high-frequency range, preferably in the range within kHz and MHz.

11. The method according to any one of claims 7 to 10, **characterized in that** in the course of the plasma treatment a hollow body (4) is treated with different plasmas that are generated due to different electromagnetic fields with respect to field strength and/or frequency.

12. The method according to any one of claims 7 to 11, **characterized in that** the electromagnetic field is varied by one or a plurality of magnets in the tubular counter electrode (8).

13. The method according to any one of claims 7 to 12, **characterized in that** the process gas or the process gases are introduced into the hollow body (4) through one or a plurality of openings (18), positioned along the longitudinal axis of the tubular counter electrode (8).

14. The method according to any one of claims 7 to 13, **characterized in that** the tubular counter electrodes (8) are heated when the plasma treatment is carried out.

15. The method according to any one of claims 7 to 14, **characterized in that** the plasma treatment is a PECVD process for the inner coating of the hollow bodies, particularly with a SiOx layer.

## Revendications

1. Dispositif pour le traitement au plasma de corps creux (4) comprenant une chambre de traitement en dépression (2) et des moyens pour produire le plasma, **caractérisé en ce que** les moyens pour produire le plasma comprennent une électrode (6) sensiblement en forme de U en section transversale, qui est agencée dans la chambre de traitement en dépression (2), le dispositif étant conçu de manière telle, que les corps creux (4), pendant l'exécution du traitement au plasma, soient plongés au moins partiellement dans l'électrode (6) en forme de U, et soient déplacés, au moins temporairement, par rapport à l'électrode (6) en forme de U.

2. Dispositif selon la revendication 1, **caractérisé en ce que** le dispositif comprend au moins deux dispositifs d'aspiration séparés, un dispositif d'aspiration (14) engendrant une première pression réduite (P2) à l'intérieur des corps creux (4), et un dispositif d'aspiration séparé (12) engendrant une deuxième pression réduite (P1) à l'intérieur de la chambre de traitement en dépression (2), mais à l'extérieur des corps creux (4), et les dispositifs d'aspiration (12, 14) étant conçus pour que la première pression réduite (P2) soit plus faible, de préférence au moins 10 à 2000 fois plus faible que la deuxième pression réduite (P1).

3. Dispositif selon l'une des revendications 1 à 2, **caractérisé en ce que** les moyens pour produire le plasma comprennent un générateur (10) pour produire un champ électromagnétique dans le domaine des hautes fréquences, de préférence dans le domaine des KHz jusqu'au domaine des MHz.

4. Dispositif selon l'une des revendications 1 à 2, **caractérisé en ce que** les moyens pour produire le plasma comprennent une ou plusieurs électrodes conjuguées (8) de forme tubulaire, qui, pendant l'exécution du traitement au plasma, se trouvent au moins temporairement à l'intérieur des corps creux (4), et les électrodes conjuguées (8) de forme tubulaire présentant le long de leur axe longitudinal, plusieurs ouvertures (18) destinées à l'introduction de gaz dans les corps creux (4).

5. Dispositif selon la revendication 4, **caractérisé en ce que** l'électrode conjuguée (8) de forme tubulaire comporte, au moins dans une zone, de préférence l'extrémité dirigée vers le fond du corps creux, au moins un aimant.

6. Dispositif selon l'une des revendications 1 à 5, **caractérisé en ce que** l'électrode (6) en forme de U comprend plusieurs segments individuels (6a-d).

7. Procédé pour le traitement au plasma de corps creux (4), d'après lequel les corps creux (4) sont transférés dans une chambre de traitement en dépression (2) dans laquelle est effectué le traitement au plasma, et le plasma est engendré par un champ électromagnétique, **caractérisé en ce que** les corps creux (4), pendant l'exécution du traitement au plasma, sont plongés au moins partiellement dans la zone intérieure d'une électrode (6) en forme de U produisant le champ électromagnétique, et sont déplacés, au moins temporairement, par rapport au champ électromagnétique.

8. Procédé selon la revendication 7, comprenant les étapes suivantes :
- introduction d'une électrode conjuguée (8) de forme tubulaire à l'intérieur des corps creux (4) ;
- établissement d'une pression appropriée dans les corps creux (4) et dans la chambre de traitement en dépression (2) ;
- addition d'un ou de plusieurs gaz de processus, et production d'un champ électromagnétique,
- production d'un plasma à l'intérieur des corps creux (4) de manière à ce que les corps creux (4), pendant le traitement au plasma, se trouvent au moins temporairement dans la zone intérieure de l'électrode (6) en forme de U, et que les corps creux (4) soient déplacés par rapport à l'électrode (6) en forme de U.

9. Procédé selon l'une des revendications 7 à 8, **caractérisé en ce qu'**à l'intérieur des corps creux (4) on établit une première pression réduite (P2), et à l'intérieur de la chambre de traitement en dépression (2), mais à l'extérieur des corps creux, on établit une deuxième pression réduite (P1), la première pression réduite (P2) étant plus faible, de préférence au moins 10 à 2000 fois plus faible que la deuxième pression réduite (P1).

10. Procédé selon l'une des revendications 7 à 9, **caractérisé en ce que** le plasma est produit par un champ électromagnétique dans le domaine des hautes fréquences, de préférence dans le domaine des KHz jusqu'au domaine des MHz.

11. Procédé selon l'une des revendications 7 à 10, **caractérisé en ce qu'**au cours du traitement au plasma, un corps creux (4) est traité avec des plasmas différents, qui sont produits en raison de champs électromagnétiques différents quant à l'intensité de champ et/ou à la fréquence.

12. Procédé selon l'une des revendications 7 à 11, **caractérisé en ce que** le champ électromagnétique est influencé par un ou plusieurs aimants dans l'électrode conjuguée (8) de forme tubulaire.

13. Procédé selon l'une des revendications 7 à 12, **caractérisé en ce que** l'addition du gaz de processus ou des gaz de processus dans le corps creux (4) s'effectue par l'intermédiaire d'une ou de plusieurs ouvertures (18), qui se trouvent le long de l'axe longitudinal de l'électrode conjuguée (8) de forme tubulaire.

14. Procédé selon l'une des revendications 7 à 13, **caractérisé en ce que** l'électrode conjuguée (8) de forme tubulaire est chauffée pendant l'exécution du traitement au plasma.

15. Procédé selon l'une des revendications 7 à 14, **caractérisé en ce que** le traitement au plasma est un processus PECVD (dépôt chimique en phase vapeur assisté par plasma) pour le revêtement intérieur des corps creux, avec en particulier une couche de SiOx.
